**Europäisches Patentamt**

**European Patent Office**

**Office européen des brevets**

(19)

(11) Veröffentlichungsnummer : **0 346 501 B1**

(12) ## EUROPÄISCHE PATENTSCHRIFT

(45) Veröffentlichungstag der Patentschrift :
**29.07.92 Patentblatt 92/31**

(51) Int. Cl.$^5$ : **A61K 37/02**

(21) Anmeldenummer : **89901342.9**

(22) Anmeldetag : **14.12.88**

(86) Internationale Anmeldenummer :
**PCT/SU88/00255**

(87) Internationale Veröffentlichungsnummer :
**WO 89/06134 13.07.89 Gazette 89/15**

(54) **ARZNEIMITTELZUBEREITUNG ZUR BEHANDLUNG DES IMMUNMANGELS.**

(30) Priorität : **30.12.87 SU 4352833**

(43) Veröffentlichungstag der Anmeldung :
**20.12.89 Patentblatt 89/51**

(45) Bekanntmachung des Hinweises auf die
Patenterteilung :
**29.07.92 Patentblatt 92/31**

(84) Benannte Vertragsstaaten :
**AT BE CH DE FR GB IT LI NL SE**

(56) Entgegenhaltungen :
**EP-A- 0 240 033**
**SU-A- 1 277 903**
**US-A- 4 699 898**
**US-A- 4 751 216**
**Keine weiteren Entgegenhaltungen.**

(73) Patentinhaber : **VSESOJUZNY
KARDIOLOGICHESKY NAUCHNY TSENTR
AKADEMII MEDITSINSKIKH NAUK SSSR**
**ul. 3 Cherepkovskaya, 15a**
**Moscou, 121552 (SU)**

(72) Erfinder : **YAKOVLEV, German Mikhailovich**
**ul. Komissara Smirnova, 8-50**
**Leningrad, 194175 (SU)**
Erfinder : **MOROZOV, Vyacheslav Grigorievich**
**ul. Korablestroitelei, 38-3-194**
**Leningrad, 199155 (SU)**
Erfinder : **KHAVINSON, Vladimir
Khatskelevich**
**pr. Entuziastov, 28-1-43**
**Leningrad, 195279 (SU)**
Erfinder : **DEIGIN, Vladislav Isakovich**
**ul. Osennaya, 4-1-276**
**Moscow, 121609 (SU)**
Erfinder : **KOROTKOV, Andrei Marxovich**
**ul. Osennaya, 4-1-44**
**Moscow, 121609 (SU)**

(74) Vertreter : **von Füner, Alexander, Dr. et al
Patentanwälte v. Füner, Ebbinghaus, Finck
Mariahilfplatz 2 & 3
W-8000 München 90 (DE)**

## Beschreibung

Die vorliegende Erfindung bezieht sich auf ein. Gebiet der Pharmacie und betrifft insbesondere ein neues Arzneimittel zur Behandlung der immuninsuffizienten Zustände.

Zugrundeliegender Stand der Technik

Es sind Arzneimittel zur Behandlung der immuninsuffizienten Zustände, insbesondere Thymuspräratate bekannt, die aus dem tierischen Rohstoff, Thymosin der Fraktion 5 erhalten werden (Goldstein A. L., Guha A., Latz M. M., Hardy H. A., White A., Proc. Nat. Acad. Sci, USA, 1972, v, 69, p. 1800-1803), Thymalin (CH, A, 659586), Taktivin (US, A, 4377511). Diese Präparate bestehen aus einem Komplex der Stoffe der Polypeptidnatur, die fähig sind, verschiedene Stufen der Proliferation und Differentiation der T-Lymphozyten zu regeln. Die praktische Verwendung solcher Präparate ist jedoch wesentlich erschwert, weil das Verfahren zu deren Herstellung kompliziert ist, die Ausbeute an Wirkstoffen niedrig ist, deren physikalisch-chemische und biologische Eigenschaften variabel sind. Außerdem entstehen bei den Kranken manchmal Nebenreaktionen bei ihrer Verwendung, weil in den natürlichen Thymuspräparaten Ballastkomponenten vorliegen.

Eines der effektivsten Mittel zur Behandlung der immuninsuffizienten Zustände ist Thymalin, das einen Komplex der Polypeptide von 600 bis 6000 D Molekularmasse in einem pharmazeutischen Lösungsmittel, beispeilsweise Glyzin darstellt, Es wird in einer Dosis von 10 mg Wirkstoff einmal täglich innerhalb von 5 Tagen eingeführt. Der Gehalt an Wirkkomponenten im gegebenen Präparat bedingt dessen Verwendung in hoben Dosen (bis 50 mg pro eine Behandlungskur).

Das Ziel der vorliegenden Erfindung besteht in der Entwicklung solch eines Arzneimittels zur Behandlung der immuninsuffizienten Zustände, das eine hohe Wirksamkeit bei einer niedrigen Toxizität besitzt und keine Nebenwirkungen hervorruft.

Der Erfindung liegt die Aufgabe zugrunde, ein Arzneimittel sur Behandlung der immuninsuffizienten Zustände zu entwickeln, das solch einen Wirkstoff der Peptidnatur enthält, der die Erhöhung der biologischen Wirksamkeit der Präparats, eine niedrige Toxizität bewirkt und keine Nebeneffekte hervorruft.

Diese Aufgabe wird dadurch gelöst, daß das Arzneimittel zur Behandlung der immuninsuffizienten Zustände, das einen Wirkstoff der Peptidnatur und ein pharmazeutisches Verdünnungsmittel vorsieht, erfindungsgemäß als Wirkstoff der Peptidnatur ein Peptid folgender Struktur enthält:

H-L-GLU-L Trp-OH.

Das erfindungsgemäße Arzneimittel zur Behandlung der immuninsuffizienten Zustände besitzt eine hohe Wirksamkeit in einer Dosis, die um das 50-500fache niedriger als die von Thymalin ist, es besitzt eine ausgeprägte immunostimulierende Wirkung bei der Radiationsimmunoinsuffizienz. Unter Einwirkung der Arzneimittels wird eine signifikante Stimulierung der Produktion von lymphozyten und T-Lymphozyten sowie eine ausgeprägte Modulation der Verhältnisses zwischen den Subpopulationen der immukompetenten Zellen nachgewiesen.

Es gelang nicht, $LD_{50}$ der erfindungsgemäßen Arzneimittels festzustellen, weil bei einer 1000maligen Erhöhung der therapeutischen Dosis (1 mg/kg) der Letalausgang bei keinem Tier nachgewiesen wurde.

Das erfindungsgemäße Präparat ruft keine Nebenwirkungen hervor.

Erfindungsgemäß ist es zweckmäßig, daß das Arzneimittel in Form einer Injektionslösung den Wirkstoff in einer Menge von 0,001 bis 0,01 Gew.-% enthält.

Erfindungsgemäß ist es zweckmäßig, daß das Arzneimittel als pharmazeutisches Verdünnungsmittel eine 0,9%ige wäßrige Natriumchloridlösung bzw. eine 0,5%ige Novokainlösung enthält,

Erfindungsgemäß ist es zweckmäßig, daß das Arzneimittel in Form von Tabletten, Suppositorien oder Kapseln den Wirkstoff stoff in einer Menge von 0,1 mg pro eine Tablette, ein Suppositorium, eine Kapsel enthält.

Es ist zweckmäßig, daß das Arzneimittel in Form von Tabletten, Suppositorien oder Kapseln erfindungsgemäß als pharmazeutisches Verdünnungsmittel ein Füllmittel - Stärke Glukose, Glyzin enthält.

Andere Ziele und Vorteile der erfindungsgemäßen Erfindung sind aus der nachfolgenden ausführlichen Beschreibung des Arsneimittels zur Behandlung der immuninsuffizienten Zustände verständlich.

Das erfindungsgemäße Arzneimittel zur Behandlung der immuninsuffizienten Zustände enthält das Peptid folgender Struktur H-L-Glu-L-Trp-OH und ein pharmazeutisches Verdünnungsmittel.

Das genannte Peptid kann nach der bekannten zweistufigen chemischen Synthese in der Lösung erhalten werden, ausgehend vom Aktivester des geschützten L-Glutaminsäure- und L-Tryptophan-Derivats. Nach der Erfindung der Schutzgruppen und der chromatographischen Reinigung wird das Peptid lyophilisiert und in Form von einem amorphen Pulver erhalten.

Das Peptid der Struktur H-L-Glu-Trp-OH stellt ein weißes lyophilisiertes Pulver dar, das in Wasser, Dime-

2

thylformanid leicht löslich, in Chloroform, Äther unlöslich ist. $/\alpha/_D^{22}$ = 12,6; C=0,5, $H_2O$ .$R_f$= 0,65 (Butanol:Essigsäure: Wasser= 3:1:1). Das Adsorptionsmaximum im UV-Bereich beträgt 275±5 nm. Im H'-NMR-Spektrum sind bei 500 MHz 0,001 Mol/l Peptidlösung folgende Signale entsprechender Aminosäurenreste vorhanden.

Trp - 3,17; 3,37; 4,57; 7,16; 7,24; 7,71; 7,49;

Glu - 1,90; 1,96; 2,21; 3,72.

Die angeführten Angaben bestätigen eindeutig die Struktur der synthetisierten Verbindung - des Wirkstoffes des erfindungsgemäßen Arzneimittels.

Der Wirkstoff des erfindungsgemäßen Arzneimittels kann sowohl in Form vom freien Peptid als auch in Form von wasserlöslichen Salzen, beispielsweise Natrium-, Kalium-, Ammonium-, Zinksalzen verwendet werden.

Das erfindungsgemäße Präparat kann in verschiedenen galenischen Formen, beispeilsweise in Tabletten, Lösungen (intranasale und Injektionslösungen), Tropfen, Salben, Suppositorien Verwendung finden. Vorzugsweise wird das erfindungsgemäße Präparat in Form von Injectionslösungen mit einem Gehalt an Wirkstoff von 0,001 bis 0,01 Gew.-% (0,0001 bis 0,001 mg/kg Körpermasse oder 10 bis 100 μg Wirkstoff in 1 ml Lösungsmittel) verwendet. Als pharmazeutisches Verdünnungsmittel für die Injektionsform enthält das Präparat ein beliebiges pharmazeutisch geeignetes Lösungsmittel, beispielsweise die 0,9%ige wäßrige Natriumchloridlösung, destilliertes Wasser, Novokainlösung für Injektionen, Ringersche Lösung, Glukoselösung.

Das erfindungsgemäße Präparat in Form von Tabletten und Suppositorien enthält den Wirkstoff vorzugsweise in einer Menge von 0,01 mg pro eine Tablette oder ein Suppositorium. Als pharmazeutisches Füllmittel für Tabletten ist es zweckmäßig Stärke, Glukose, Glyzin auszunuten. Als pharmazeutisches Füllmittel für Suppositorien kann eine beliebige pharmazeutisch geeignete Grundlage ausgenutzt werden.

Das erfindungsgemäße Arzneimittel legt eine hohe Effektivität bei der Behandlung der immuninsuffizienten Zustände an den Tag, besitzt eine starke Wirkung auf die immunologische Reaktivität des Organismus, trägt zu einer bedeutenden Stimulierung der Produktion von Lymphozyten und T-Lymphozyten und zu einer ausgeprägten Modulation des Verhältnisses zwischen den Subpopulationen der immunkompetenten Zellen bei.

Das Präparat weist keine Toxizität auf, wird in kleinen Einzel- und Kurdosen eingeführt, besitzt eine große therapeutische Wirkungsbreite. Das erfindungsgemäße Präparat kann eine breite Verwendung in der medizinischen Praxis zur Behandlung un Prophylaxe einer Reihe von Erkrankungen des Menschen finden.

Das erfindungsgemäße Präparat zur Behandlung der immuninsuffizienten Zustände wurde im Tierversuch und in der Klinik an Menschen geprüft.

Die biologische Wirksamleit des erfindungsgemäßen Prärarats auf Grundlage von Dipeptid wurde im Vergleich zur Wirksamkeit von Thymalin bei sekunädren immuninsuffizienten Zuständen untersucht, die insbesondere durch die Bestrahlung indiziert wurden. Die Versuche wurden an männlichen Meerschweinchen von 150 bis 200 g Körpermasse angestellt. Die Tiere wurden in der Anlage mit einer Dosis von 1 Gr bestrahlt. Einen Tag nach der Bestrahlung wurden den Versuchsgruppen (bis 10 Meerschweinchen in jeder Gruppe) Thymalin und das erfindungsgemäße Präparat in einer Dosis von 0,0001 bis 0,001 mg/kg in 0,5 ml 0,9%ige wäßrige Natriumchloridlösung intramuskulär innerhalb von 3 Tagen eingeführt. Die Kontrolltiere erhielten zu 0,5 ml 0,9%ige wäßrige Natriumchloridlösung nach dem ähnichen Schema. 10 Tage nach der Bestrahlung wurde im Thymus und in der Milz die Anzahl von Karyozyten, T- und B-Lymphozyten nach der Methode der Rosettenbildung bestimmt.

Die erhaltenen Ergebnisse sind in Tabellen 1 und 2 angeführt.

Es wurde festgestellt, daß Dipeptid in einer Dosis von 0,001 mg/kg eine immunostimulierende Wirkung ausübt, indem es mehr als um das 3fache die Anzahl der Karyozyten und T-Lymphozyten im Thymus der bestrahlten Tiere erhöht. Gleichzeitig normalisiert Dipeptid in einer Dosis von 0,001 mg/kg den B-Lymphozytengehalt im Thymus und in der Milz der bestrahlten Tiere. Thymalin übte in allen untersuchten Dosen keinen gesicherten Einfluß auf den Zellenbestand des Thymus und der Milz der bestrahlten Tiere aus.

Nach den Ergebnissen, die in der zusätzlichen Versuchsserie unter ähnlichen Bedingungen erhalten wurde, wurde festgestellt, daß Thymalin eine immunostimulierende Wirkung nur in einer Dosis von 0,1 mg/kg Körpermasse des Tiers ausübt (Tab. 3).

Tabelle 1.

Vergleichende Untersuchung der Wirkung des erfindungsgemäßen Präparats und von Thymalin auf den Zellenbestand des Thymus der bestrahlten Meerschweinchen ($X \pm m$)

| Tiergruppe | Karyozytenzahl (T/mg) | T-Lymphozytenzahl (T/mg) | B-Lymphozytenzahl (T/kg) |
|---|---|---|---|
| Intakte Kontrolle (Bestrahlung) | $481,4 \pm 21,3$ | $378,5 \pm 25,5$ | $4,8 \pm 0,3$ |
| lung) | $87,4 \pm 13,2$ | $49,6 \pm 5,6$ | $5,7 \pm 0,6$ |
| Dipeptid (0,00001 mg/kg) | $89,3 \pm 10,7$ | $51,3 \pm 6,7$ | $5,3 \pm 0,5$ |
| Dipeptid (0,0001 mg/kg | $139,8 \pm 14,1^x$ | $89,4 \pm 9,1^x$ | $4,9 \pm 0,5^x$ |
| Dipeptid (0,001 mg/kg) | $275,8 \pm 26,4^x$ | $194,7 \pm 21,3^x$ | $3,3 \pm 0,4^x$ |
| Thymalin (0,00001 mg/kg) | $85,4 \pm 11,6$ | $53,6 \pm 7,2$ | $5,4 \pm 0,6$ |
| Thymalin (0,0001 mg/kg | $90,4 \pm 9,7$ | $46,3 \pm 5,7$ | $5,3 \pm 0,5$ |
| Thymalin (0,001 mg/kg | $89,1 \pm 13,6$ | $47,9 \pm 6,1$ | $5,7 \pm 0,7$ |

x - statistisch gesichert ($P < 0,05$) im Vergleich zur Kontrolle

Tabelle 2.

Vergleichende Untersuchung der Wirkung des erfindungsgemäßen Präparats und des Thymalins auf den Zellenbestand der Milz der bestrahlten Meerschweinchen ($X \pm m$)

| Tiergruppe | Karyozytenzahl (T/mg) | T-Lymphozyten-zahl (T/mg) | B-Lymphozyten-zahl (T/mg) |
|---|---|---|---|
| Intakte Kontrolle | $409,3 \pm 25,6$ | $40,4 \pm 2,9$ | $72,5 \pm 5,4$ |
| (Bestrahlung) | $147,9 \pm 23,2$ | $29,8 \pm 3,4$ | $51,4 \pm 4,3$ |
| Das erfindungsgemäße Präparat (0,00001 mg/kg) | $150 \pm 15,8$ | $31,2 \pm 3,6$ | $50,9 \pm 5,4$ |
| Das erfindungsgemäße Präparat (0,0001 mg/kg) | $148,3 \pm 16,0$ | $30,4 \pm 3,9$ | $62,4 \pm 6,1^{x}$ |
| Das erfindungsgemäße Präparat (0,001 mg/kg) | $146,6 \pm 18,1$ | $29,9 \pm 3,3$ | $73,2 \pm 5,1^{x}$ |
| Thymalin (0,00001 mg/kg) | $151,4 \pm 16,7$ | $32,0 \pm 4,1$ | $53,7 \pm 6,3$ |
| Thymalin (0,0001 mg/kg | $147,3 \pm 15,1$ | $31,7 \pm 5,2$ | $49,7 \pm 5,8$ |
| Thymalin (0,001 mg/kg | $150,0 \pm 16,6$ | $28,9 \pm 4,1$ | $54,7 \pm 5,2$ |

x - statistisch gesichert ($P < 0,05$) im Vergleich zur Kontrolle

## Tabelle 3

Einfluß von Thymalin auf den Zellenbestand des Thymus und der Milz der bestrahlten Meerschweinchen (X±m)

| Tiergruppe | Karyozytenzahl (T/mg) | |
|---|---|---|
| | Thymus | Milz |
| I | 2 | 3 |
| Intakte | 481,4±21,3 | 409,3±25,6 |
| Kontrolle (Bestrahlung) | 87,4±13,2 | 147,9±23,2 |
| Thymalin (0,01 mg/kg) | 89,3±11,6 | 153,6±17,1 |
| Thymalin (0,05 mg/kg) | 101,4±15,3 | 149,4±16,0 |
| Thymalin (0,1 mg/kg) | $280,7±27,3^x$ | 154,3±16,1 |

Fortsetzung der Tabelle 3

| Tiergruppe | Karyozytenzahl (T/mg) | | | |
|---|---|---|---|---|
| | Thymus | Milz | Thmus | Milz |
| I | 2 | 3 | 4 | 5 |
| Intakte | 378,5±25,5 | 40,4±2,9 | 4,8±0,3 | 72,5±5,4 |
| Kontrolle (Bestrahlung | 49,6±5,6 | 29,8±3,4 | 5,7±0,6 | 51,4±4,3 |
| Thymalin (0,01 mg/kg) | 51,3±7,8 | 33,6±5,7 | 5,9±0,7 | 54,5±6,3 |
| Thymalin (0,05 mg/kg) | 73,7±14,1 | 26,4±4,1 | 5,1±0,6 | 61,7±9,0 |
| Thymalin (0,1 mg/kg | $206,7±19,8^x$ | 30,1±3,3 | $3,9±0,5^x$ | $75,8±6,2^x$ |

x - statistisch gesichert ($P < 0,05$) im Vergleich zur Kontrolle

Aus diese Weise besitzt das erfindungsgemäße Präparat in einer um das 50-500fache niedrigeren Dosis als Thymalin eine ausgeprägte immunostimulierende Wirkung bei der Radiationsimmuninsuffizienz.

Es wurde eine vergleichende Einschätzung des Einflusses der erfindungsgemäßen Präparats und des Thymalins auf die Expression der Rezeptoren der von inakten Meerschweichen erhaltenen Thymozyten durchgeführt. Es is bekannt, daß nach der Bearbeitung der Lymphozyten mit Trypsin die Zerstörung des großen Teils der E-Rezeptoren auf der Zellenoberfläche vor sich geht; infolgedessen sinkt der Prozentgehalt an T-lymphozyten, der in der Reaktion der Rosettenbildung festgestellt wird (in Englisch bekannt auch als E-RFC). Die Zugabe der Thymuspräparate zu den mit Trypsin behandelten Lymphozyten führt zur Wiederherstellung der zerstörten E-Rezeptoren, was sich in der Vergrößerung des Prozentgehalts an E-RFC (T-Lymphozyten) äußert.

Das erfindungsgemäße Präparat und Thymalin wurden den von 10 inkaten Meerschweinchen erhaltenen Thymozyten in Dosen von 0,0001 bis 1 µg/ml zugesetzt. Die Untersuchungsergebnisse sind in der Tabelle 4

6

EP 0 346 501 B1

angeführt.

Tabelle 4

Einfluß des erfindungsgemäßen Präparats und des Thymalin
auf die Expression der R-Rezeptoren von Thymozyten ($X \pm m$)

| Präparat | Ea – RFC (%) | P |
|---|---|---|
| 1 | 2 | 3 |
| Kontrolle | $36,1 \pm 3,1$ | – |
| Das erfindungsgemäße Präparat (0,0001 ug/ml) | $37,4 \pm 4,0$ | $> 0,05$ |
| Das erfindungsgemäße Präparat (0,001 µg/ml) | $47,4 \pm 5,1$ | $> 0,05$ |
| Das erfindungsgemäße Präparat (0,01 µg/ml) | $61,4 \pm 5,3$ | $< 0,05$ |
| Das erfindungsgemäße Präparat (1 ug/ml) | $62,7 \pm 6,1$ | $< 0,05$ |
| Thymalin (0,0001 µg/ml) | $35,3 \pm 3,9$ | $> 0,05$ |
| Thymalin (0,001 µg/ml) | $39,6 \pm 4,1$ | $> 0,05$ |
| Thymalin (0,01 µg/ml) | $36,7 \pm 4,0$ | $> 0,05$ |
| Thymalin (1 µg/ml) | $57,0 \pm 4,7$ | $< 0,05$ |

Es wurde festgestellt, daß sowohl das erfindungsgemäße Präparat als auch Thymalin E-Rezeptoren bei den im Trypsin behandelten Thymozyten wiederherstellten. Das erfindungsgemäße Präparat ist in Dosen von 0,01 bis 1 µg/ml effektiv.

Es wurde die Einschätzung der Flinisch-immunologischen Effectivität des erfindungsgemäßen Arzneimittels bei 300 Kranken mit eitrigen Entzündungserkrankungen der Knochen und der Weichgewebe durchgeführt (chronische post-traumatische Osteomyelitis der langen röhrenförmigen Knochen, eitrige Entzündungskomplikationen des Traumas und der operativen Eingriffe bei den Erkrankungen des Stütz-Bewegungsapparats, akute und chronische eitrige Entzündungserkrankungen der Knochen und Weichgewebe der Kiefer-Gesichtsgegend, Staphylokokkuspyodermien).

Das erfindungsgemäße Arzneimittel wurde intramuskulär oder intranasal zu je 100 µg einmalig innerhalb von 3-10 Tagen (durchschnittlich 500 µg Präparat pro eine Behandlungskur) eingeführt. Die Kontrollgruppe bildeten 200 Kranke mit ähnlicher Pathologie.

Bei den Kranken mit den eitrigen Entzündungserkrankungen der Knochen und Weichgewebe verschiedener Lokalisation wurde die Herabsetzung verschiedenen Grades von quantitativen und insbesondere funktionellen Indexe der Zellenimmunität und der unspezifischen Reistenz an den Tag gelegt. Ls wurde auch die Störung der Differenzierung der Subpopulationen der T-Zellen: die Verminderung der Anzahl der T-Helperzellen (OKT4[+]) und der T-Supressoren (OKT8[+]) nachgewiesen.

Die Verwendung des erfindungsgemäßen Arzneimittels in der komplexen Behandlung trug zur Besserung des klinischen Krankheitsverlaufs oder zur Ausheilung bei 75,3 bis 91,7% der Kranken bei, was sich in der Verminderung der Zeit der Wundenreinigung und -heilung, in der Verminderung der Fläche der Destruktion des Knochengewebes, in einem schnelleren Kupieren der eitrig-entzündlichen Hauterkrankungen, in der Herabsetzung der Behandlungsdauer um 20 bis 30 % im Vergliech zu den Kontrollkranken äußerte. Die klinische Effektivität des erfindungsgemäßen Arzneimittels wurde durch die Wiederherstellung der quantitativen und funktionellen Indexe des T-Systems der Immunität und der unspezifischen Resistenz begleitet.

Im keinem Fall der klinischen Anwendung des erfindungsgemäßen Präparats wurden toxische und allergische Reaktionnen verzeichnet.

7

Es wurde die vergleichende Einschätzung des Einflusses des erfindungsgemäßen Präparats und des Thymalins auf die Subpopulationen der Lymphozyten bei den Kranken bei verschiedenen Pathologien durchgeführt, und zwar: auf die T-Helperzellen (OKT4$^+$), T-Supressoren (OKT 8$^+$), den Differenzierungskoeffizienten OKT 4$^+$/OKT 8$^+$ und B-Lymphozyten (Ig$^+$) bei den Kranken an Thymomegalie, Thymektomie, Infektarthritis, Furunkulose und Mammakarzinom (nach der Strahlentheratpe). In den Lymphozytenkulturen des peripheren Bluts der Kranken wurde nach der Immunofluoreszenzmethode mit Hilfe der monoklonalen Antikörper der Prozentgehalt an Subpopulationen der Lymphozyten vor und nach dem Eintragen in die Zellkulturen von Thymalin und des erfindungsgemäßen Präparats in den effektivsten Dosen - 1 µg/ml bzw. 0,01 µg/ml bestimmt.

Die Untersuchungsergebnisse sind in Tabellen 5 und 6 dargestellt.

Tabelle 5

Einfluß von Thymalin auf die Subpopulationen der Lymphozyten bei den Kranken bei verschiedener Pathologie (im Zähler - die Ausgangszahl, im Nenner - nach der Zugabe von Thymalin)

| Pathologie | Zahl der Versuche | Lymphozytenzahl (%) $\overline{X}{\pm}m$ | |
| --- | --- | --- | --- |
| | | T-Helperzellen (OKT 4$^+$) | T-Supressoren (OKT 8$^+$) |
| 1 | 2 | 3 | 4 |
| Gesunde | 18 | 35,3$\pm$ 2,7 | 21,3$\pm$0,9 |
| | | 38,2$\pm$2,9 | 18,8$\pm$0,8 |
| Thymomegalie | 8 | 23,4$\pm$1,7 | 8,2$\pm$1,0 |
| | | 32,6$\pm$2,8$^X$ | 19,7$\pm$1,7$^X$ |
| Thymektomie | 4 | 10,5$\pm$1,3 | 14,5$\pm$0,7 |
| | | 20,6+1,7$^X$ | 16,1$\pm$1,1 |

Fortsetzung der Tabelle 5

| 1 | 2 | 3 | 4 |
|---|---|---|---|
| Infektarthritis | 4 | $20,8\pm1,4$ | $26,5\pm2,1$ |
| | | $29,5\pm1,7^x$ | $22,3\pm1,7$ |
| Furunkulose | 6 | $16,6+1,2$ | $12,0+0,9$ |
| | | $26,9\pm1,1^x$ | $18,3\pm1,3^x$ |
| Mammakarzinom (nach der Strahlentherapie | 10 | $23,3\pm2,1$ | $21,3\pm1,7$ |
| | | $32,6\pm3,4^x$ | $19,6\pm1,6$ |

Fortsetzung der Tabelle 5

| Pathologie | Anzahl der Versuche | Lymphozytenzahl (%) $X \pm m$ | |
|---|---|---|---|
| | | $OKT\ 4^+$ / $OKT\ 8^+$ | B-Lymphozyten $(Ig^+)$ |
| 1 | 2 | 5 | 6 |
| Gesunde | 18 | $1,66\pm0,13$ | $13,8\pm1,2$ |
| | | $2,03\pm0,16^x$ | $12,1\pm1,1$ |
| Thymomegalie | 8 | $2,85\pm0,19$ | $16,4\pm1,3$ |
| | | $1,65\pm0,11^x$ | $11,4\pm1,1$ |
| Thymektomie | 4 | $0,72\pm0,08$ | $12,5\pm1,0$ |
| | | $1,28\pm0,11^x$ | $17,7\pm1,3^x$ |
| Infektarthritis | 4 | $0,78\pm0,09$ | $8,9\pm0,9$ |
| | | $1,32\pm0,11^x$ | $14,3\pm1,2$ |
| Furunkulose | 6 | $1,38\pm0,82$ | $19,9\pm1,7$ |
| | | $1,47\pm1,2^x$ | $15,6\pm1,2$ |
| Mammakarzinom (nach der Strahlentherapie) | 10 | $1,09\pm0,04$ | $15,6\pm1,2$ |
| | | $1,65\pm0,09^x$ | $13,6\pm1,0$ |

x - statistisch gesichert ($P < 0,05$) im Vergleich zu den Ausgangsangaben

Tabelle 6.

Einfluß des erfindungsgemäßen Arzneimittels auf die Subpopulationen der Lymphozyten bei den Kranken mit verschiedener Pathologie (im Zähler - die Ausgangszahl, im Nenner - nach der Zugabe des erfindungsgemäßen Arzneimittels)

| Pathologie | Anzahl der Versuche | Lymphozytenzahl (%), $X \pm m$ | |
|---|---|---|---|
| | | T-Helperzellen (OKT $4^+$) | T-Supressoren (OKT $8^+$) |
| 1 | 2 | 3 | 4 |
| Gesunde | 18 | $35,4 \pm 2,7$ / $37,6 \pm 2,3$ | $21,3 \pm 0,9$ / $19,4 \pm 0,9$ |
| Thymomegalie | 8 | $23,4 \pm 1,7$ / $33,4 \pm 2,6^X$ | $8,2 \pm 1,0$ / $18,3 \pm 1,6^X$ |
| Thymektomie | 4 | $10,5 \pm 1,3$ / $21,1 \pm 1,5^X$ | $14,6 \pm 0,7$ / $15,3 \pm 1,3$ |
| Infektarthritis | 4 | $20,8 \pm 1,4$ / $30,1 \pm 1,9^X$ | $26,5 \pm 2,1$ / $24,7 \pm 2,0$ |
| Furunkulose | 6 | $16,6 \pm 1,2$ / $27,4 \pm 1,2^X$ | $12,0 \pm 0,9$ / $19,1 \pm 1,4^X$ |
| Mammakarzinom (nach der Strahlentherapie) | 10 | $23,3 \pm 2,1$ / $31,7 \pm 2,9^X$ | $21,3 \pm 1,7$ / $18,6 \pm 1,9$ |

Fortsetzung der Tabelle 6

| Pathologie | Anzahl der Versuche | Lymphozytenzahl (%),X± m | |
|---|---|---|---|
| | | OKT 4⁺ / OKT 8⁺ | B-Lymphozyten (Ig⁺) |
| 1 | 2 | 5 | 6 |
| Gesunde | 18 | 1,66±0,13 | 13,8±1,2 |
| | | 1,94±0,11 | 12,6±1,3 |
| Thymomegalie | 8 | 2,85±0,19 | 16,4±1,3 |
| | | 1,83±0,12$^x$ | 12,6±1,3$^x$ |
| Thymektomie | 4 | 0,72±0,08 | 12,5±1,0 |
| | | 1,38±0,09$^x$ | 16,4±1,2$^x$ |
| Infektarthritis | 4 | 0,78±0,09 | 8,9±0,9 |
| | | 1,22±0,12$^x$ | 14,1±1,3 |
| Furunkulose | 6 | 1,38±0,62 | 19,9±1,7 |
| | | 1,43±1,3$^x$ | 16,4±1,7 |
| Mammakarzinom (nach der Strahlentherapie) | 10 | 1,09±0,04 | 15,5±1,2 |
| | | 1,70±1,5 | 14,7±1,2 |

x - statistisch gesichert (P < 0,05) im Vergleich zu den Ausgangsangaben

Es wurde festgestellt, daß Thymalin und das erfindungsgemäße Arzneimittel in einer Dosis, die ungefähr um das 100fache niedriger als die von Thymalin ist, das Verhältnis zwischen den Subpopulationen der Lymphozyten bei allen Gruppen der untersuchten Kranken normalisiert.

Somit zeigten die Ergebnisse der experimentellen Untersuchung des neuen Arzneimittels seine hohe Effektivität bei der Behandlung der immuninsuffizienten Zustände. Im Vergleich zu Thymalin besitzt das Arzneimittel eine starke (ungefähr um das 50-500fache) Wirkung auf die immunologische Reaktivität des Organismus. Unter dem Finfluß des Arzneimittels wird eine wesentliche Stimulierung der Produktion von Lymphozyten und T-Lymphozyten sowie eine ausgeprägte Modulation des Verhältnisses zwischen den Suppopulationen der immunkompetenten Zellen nachgewiesen.

Das erfindungsgemäße Arzneimittel kann in verschiedenen galenischen Formen verwendet werden: in Form von Injektionslösungen, Tabletten, Suppositorien, Salben. Beim Gebrauch des erfindungsgemäßen Arzneimittels in Form von Injektionslösungen werden die Lösungen mit einem Gehalt an Wirkstoff von 0,001 bis 0,01Gew-% (0,0001 bis 0,001 mg/kg Körpermasse oder 10 bis 100 mg Wirkstoff pro 1 ml Lösungsmittel) verwendet. Als Verdünnungsmittel enthält das Arzneimittel vorzugsweise die 0,9%ige wäßrige Natriumchloridlösung, destilliertes Wasser, die Novokainlösung für die Injektionen, die Ringersche Lösung, die Glukoselösung.

Das Arzeimittel in Form von Tabletten und Suppositorien enthält den Wirkstoff vorzugsweise in einer Menge von 0,1 mg pro eine Tablette oder ein Suppositorium. Für die Tabletten und Suppositorien wird als pharmazeutisches Füllmittel eine beliebige pharmazeutisch geeignete Grundlage verwendet. Das Arzneimittel in Form von Injektionslösung wird intramuskulär zu je eine Injektion innerhalb von 5 Tagen eingeführt. Intranasal wird das Arzneimittel auch innerhalb von 5 Tagen zu je eine Ampulle oder eine Salbentube eingeführt.

Das erfindungsgemäße Arzneimittel besitzt keine Nebenwirkung und hat keine Kontraindikationen. Die galenischen Formen des erfindungsgemäßen Arzneimittels werden nach der allgemein bekannten Methode hergestellt.

Das erfindungsgemäße Arzneimittel wird in der medizinischen Praxis zur Behandlung und Prophylase der immuninsuffizienten Zustände des Menschen verwendet.

## Patentansprüche

1. Arzneimittel zur Behandlung der immuninsuffizienten Zustände, das einem Wirkstoff der Peptidnatur und ein pharmazeutisches Verdünnungsmittel vorsieht, **dadurch gekennzeichnet,** daß es als Wirkstoff der Peptidnatur ein Peptid folgender Struktur H-L-Glu-L-Trp-Oh enthält.

2. Arzneimittel nach Anspruch 1, in Form einer Injektionslösung, **dadurch gekennzeichnet,** dass es den Wirkstoff in einer Menge von 0,001 bis 0,01 Gew.-% enthält.

3. Arzneimittel nach Anspruch 1 bis 2, **dadurch gekennzeichnet,** daß es als pharmazeutisches Verdünnungsmittel eine 0,9%ige wäßrige Natriumchloridlösung bzw. eine 0,5%ige wäßrige Novokainlösung enthält.

4. Arzneimittel nach Anspruch 1 in Form von Tabletten, Suppositorien oder Kapseln, **dadurch gekennzeichnet,** daß es den Wirkstoff in einer Menge von 0,1 mg pro eine Tablette, ein Suppositorium oder eine Kapsel enthält.

5. Arzneimittel nach Anspruch 4, **dadurch gekennzeichnet,** daß es als pharmazeutisches Verdünnungsmittel den Füllstoff -- Stärke, Glukose, Glyzin enthält.

## Claims

1. A medicament for the treatment of immune-deficient states, which provides an active substance of peptide nature and a pharmaceutical thinning agent, **characterised in that,** as the active substance of peptide nature, it contains a peptide of the following structure H-L-Glu-L-Trp-OH.

2. A medicament according to Claim 1, in the form of an injection solution, **characterised in that** it contains the active substance in a quantity of 0.001 to 0.01 % by weight.

3. A medicament according to Claims 1 to 2, **characterised in that,** as the pharmaceutical thinning agent, it contains a 0.9% aqueous sodium chloride solution and/or a 0.5% aqueous Novocaine solution.

4. A medicament according to Claim 1 in the form of tablets, suppositories or capsules, **characterised in that** it contains the active substance in a quantity of 0.1 mg per one tablet, suppository or capsule.

5. A medicament according to Claim 4, **characterised in that,** as the pharmaceutical thinning agent, it contains the filling material -- starch, glucose, glycine.

## Revendications

1. Médicament pour le traitement des états immunodéficitaires, qui prévoit un agent actif de nature peptidique et un diluant pharmaceutique, **caractérisé en qu'**il contient, en tant qu'agent actif de nature peptidique, un peptide de la structure suivante H-L-Glu-L-Trp-OH.

2. Médicament selon la revendication 1, sous la forme d'une solution pour injection, **caractérisé en ce qu'**il contient l'agent actif en une quantité comprise entre 0,001 et 0,01% en poids.

3. Médicament selon la revendication 1 ou 2, **caractérisé en ce qu'**il contient, en tant que diluant pharmaceutique, une solution aqueuse à 0,9% de chlorure de sodium ou, respectivement, une solution aqueuse à 0,5% de novocaïne.

4. Médicament selon la revendication 1, sous la forme de comprimés, suppositoires ou capsules, **caractérisé en ce qu'**il contient l'agent actif en une quantité de 0,1 mg par comprimé, suppositoire ou capsule.

5. Médicament selon la revendication 4, **caractérisé en ce qu'**il contient, en tant que diluant pharmaceutique, les charges amidon, glucose, glycérine.